Europäisches Patentamt

European Patent Office

Office européen des brevets

(19)

(11) **EP 1 595 549 A1**

(12) **EUROPEAN PATENT APPLICATION**
published in accordance with Art. 158(3) EPC

(43) Date of publication:
**16.11.2005 Bulletin 2005/46**

(21) Application number: **04712185.0**

(22) Date of filing: **18.02.2004**

(51) Int Cl.⁷: **A61K 47/44**, A61K 9/50,
A61K 9/66, A61K 47/02,
A61K 47/10, A61K 47/14,
A61K 47/26, A61K 47/34,
A61K 47/36, A61K 47/38,
A61K 38/00, A61K 38/22,
A61K 38/27, A61P 43/00

(86) International application number:
**PCT/JP2004/001760**

(87) International publication number:
**WO 2004/073749 (02.09.2004 Gazette 2004/36)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **19.02.2003 JP 2003041323**

(71) Applicant: **Takeda Pharmaceutical Company
Limited
Osaka-shi, Osaka 541-0045 (JP)**

(72) Inventors:
• **ASAKAWA, Naoki
  c/o Takeda Pharmaceutical Comp.Ltd.
  Osaka-shi, Osaka 532-8686 (JP)**
• **HORI, Masuhisa
  c/o Takeda Pharmaceutical Comp.Ltd.
  Osaka-shi, Osaka 532-8686 (JP)**

(74) Representative: **Rickard, Timothy Mark Adrian
Takeda Euro IP Department,
11-12 Charles II Street
London SW1Y 4QU (GB)**

(54) **DISPERSANT FOR SUSTAINED RELEASE PREPARATIONS**

(57)    The present invention provides a bilayer dispersing agent for a sustained-release preparation, which contains a non water-soluble solvent and an aqueous solvent, and a sustained-release preparation wherein a microcapsule containing a physiologically active substance is dispersed in the aforementioned dispersing agent. When this preparation is subcutaneously administered, the initial release of a physiologically active substance immediately after administration is strikingly suppressed, a constant amount of a physiologically active substance is released for a long period of time from immediately after administration, superior dispersibility is afforded, and the preparation can readily pass through a needle as an injection.

**EP 1 595 549 A1**

**Description**

**Technical Field**

The present invention relates to a dispersing agent that shows a small initial release of a drug from a sustained-release preparation immediately after administration, releases a constant amount of a drug for a long period of time, and shows improved dispersibility, and a sustained-release preparation containing the same.

**Background Art**

Physiologically active peptides are known to exhibit various pharmacological actions in a living body, and are intended for use as pharmaceuticals. However, these physiologically active peptides must be administered frequently since they generally have a short half-life in a living body, therefore physical burden of patients due to the frequent injections can be considerable. For example, growth hormone (hereinafter referred to as GH), a representative hormone which is originally produced and secreted in the anterior pituitary gland, is a physiologically active peptide having widely diverse physiological activities such as growth stimulation in the body, metabolism of saccharides and lipids, anabolism of protein, and cell proliferation and differentiation. The GH has been recently produced on a large scale with *Escherichia coli* using genetic recombination technology, and put to clinical use worldwide as medicine. However, GH must be frequently administered in order to maintain an effective blood level because of its short biological half-life. Especially, in the case of patients having GH-deficient short stature, in practice GH is administered daily by subcutaneous injection to infants or young patients over a long period of time ranging from a few months to 10 years or more.

In order to deal with the problems inherent in physiologically active peptide medicine, various drug delivery systems have been studied. For example, a sustained-release agent that provides sustained-release of a physiologically active peptide for a long period is exemplified. Patent document 1 discloses a production method for a sustained-release preparation containing a water-insoluble or poorly water-soluble multivalent metal salt and a biodegradable polymer, wherein the metal salt is formed from a water-soluble peptide physiologically active substance and an aqueous solution of zinc chloride and the like. Furthermore, patent document 2 discloses a production method for a sustained-release preparation comprising adding a water-miscible organic solvent and/or a volatile salt to an aqueous solution of a physiologically active peptide, followed by lyophilizing to obtain physiologically active peptide powder, dispersing the powder in a solution of a biodegradable polymer in an organic solvent, and removing the organic solvent. Moreover, in a production method for a sustained-release microcapsule containing a physiologically active substance and a biodegradable polymer, patent document 3 discloses a production method for providing a sustained-release microcapsule which contains very little residual organic solvent and has very superior clinical characteristics as a medicine, comprising forming microcapsules and heat-drying the microcapsules at the temperature of not less than the glass transition temperature of the biodegradable polymer for about 24 to 120 hr.

Moreover, patent document 4 discloses, as a sustained-release injection, an aqueous suspension wherein a microsphere comprising a physiologically active substance (hydrophobic antipsychotic drug) included in a base comprising a biodegradable polymer is dispersed in an aqueous solvent or an oily suspension wherein the microsphere is dispersed in a vegetable oil and the like.

As the dispersing agent for dispersion liquids, (i) an aqueous suspension containing an aqueous solvent, (ii) an oil suspension containing a non water-soluble solvent and (iii) a lipid emulsion comprising a non water-soluble solvent and an aqueous solvent can be mentioned. However, an aqueous suspension shows a small effect of reducing the initial release of the drug. In addition, an oil suspension cannot readily pass through a needle in case of injection, and thus is impractical for injection. Moreover, in a lipid emulsion, a non water-soluble solvent in the form of fine oil drops constitutes a stable homogeneous system with an aqueous solvent, and therefore, the effect to reduce an initial release is small.

    Patent document 1: JP-A-8-217691 [WO96/07399]
    Patent document 2: JP-A-11-322631
    Patent document 3: JP-A-9-132524
    Patent document 4: WO94/10892 (pages 2-6)

**Disclosure of the Invention**

A sustained-release preparation is desired to maintain the activities of a drug such as a physiologically active substance while releasing a constant amount of the drug over a long period of time. Therefore, a means for suppressing the initial release immediately after administration is required. Furthermore, injections are desired to readily pass through a needle during administration, and a dispersing agent superior in dispersibility of a sustained-release preparation has been demanded.

The present inventors have conducted intensive studies to solve the above-mentioned problems, and found that, by a subcutaneous administration of a suspension obtained by dispersing with a non water-soluble solvent as a dispersing agent of a microcapsule containing a physiologically active substance, and further dispersing with an aqueous solvent (i.e., dispersing with a bilayer dispersing agent), a sustained-release preparation having very superior clinical characteristics as a medicine, in which the initial release of the physiologically active substance immediately after administration is markedly suppressed and a constant amount of physiologically active substance is released over a long period of time, can be produced unexpectedly, and that the preparation is superior in dispersibility and can readily pass through a needle as an injection, which resulted in the completion of the present invention.

Accordingly, the present invention provides

(1) a bilayer dispersing agent for a sustained-release preparation, which comprises a non water-soluble solvent and an aqueous solvent,

(2) the dispersing agent of the aforementioned (1), wherein the non water-soluble solvent is a vegetable oil,

(3) the dispersing agent of the aforementioned (2), wherein the vegetable oil is a soybean oil,

(4) the dispersing agent of the aforementioned (1), wherein the aqueous solvent is water,

(5) the dispersing agent of the aforementioned (1), wherein the volume of the aqueous solvent is about 0.2 to about 1000 times the volume of the non water-soluble solvent,

(6) the dispersing agent of the aforementioned (1), which comprises an isotonicity agent,

(7) the dispersing agent of the aforementioned (6), wherein the isotonicity agent is mannitol, sorbitol, sodium chloride, glucose or glycerol,

(8) the dispersing agent of the aforementioned (1), which comprises a surfactant,

(9) the dispersing agent of the aforementioned (8), wherein the surfactant is a nonionic surfactant,

(10) the dispersing agent of the aforementioned (1), which comprises a thickener,

(11) the dispersing agent of the aforementioned (10), wherein the thickener is carboxymethylcellulose, sodium alginate or sodium hyaluronate,

(12) the dispersing agent of the aforementioned (1), which comprises a preservative,

(13) the dispersing agent of the aforementioned (12), wherein the preservative is alkyl 4-hydroxybenzoate,

(14) the dispersing agent of the aforementioned (1), which is used for injection,

(15) a sustained-release preparation comprising a matrix containing a physiologically active substance and the dispersing agent of the aforementioned (1),

(16) the sustained-release preparation of the aforementioned (15), wherein the physiologically active substance is a physiologically active peptide,

(17) the sustained-release preparation of the aforementioned (16), wherein the physiologically active peptide has a molecular weight of about 200 to about 500,000,

(18) the sustained-release preparation of the aforementioned (16), wherein the physiologically active peptide has a molecular weight of about 5,000 to about 500,000,

(19) the sustained-release preparation of the aforementioned (16), wherein the physiologically active peptide is a hormone, a cytokine, a hematopoietic factor, a growth factor, an enzyme or an antibody,

(20) the sustained-release preparation of the aforementioned (16), wherein the physiologically active peptide is a human growth hormone,

(21) the sustained-release preparation of the aforementioned (15), wherein a base for the matrix is a biodegradable polymer,

(22) the sustained-release preparation of the aforementioned (21), wherein the biodegradable polymer is a homopolyer or copolymer of $\alpha$-hydroxycarboxylic acids or a mixture thereof,

(23) the sustained-release preparation of the aforementioned (21), wherein the biodegradable polymer is a copolymer having a composition ratio of lactic acid/glycolic acid of about 100/0 to about 40/60 mol %,

(24) the sustained-release preparation of the aforementioned (21), wherein the biodegradable polymer is a homopolymer of lactic acid,

(25) the sustained-release preparation of the aforementioned (21), wherein the biodegradable polymer has a weight-average molecular weight of about 3,000 to about 50,000,

(26) the sustained-release preparation of the aforementioned (15), wherein the matrix is a microcapsule,

(27) a production method of a sustained-release preparation, which comprises dispersing a matrix containing a physiologically active substance in the dispersing agent of the aforementioned (1),

(28) a production method of a sustained-release preparation, which comprises dispersing a matrix containing a physiologically active substance in a non water-soluble solvent, and further dispersing the obtained dispersion in an aqueous solvent, and

(29) a sustained-release preparation obtained by the method of the aforementioned (27) or (28).

The use of the dispersing agent for a sustained-release preparation of the present invention as a dispersing agent for a microcapsule containing a physiologically active substance provides effects in that the initial release of a physiologically active substance immediately after administration is strikingly suppressed, a constant amount of a physiologically active substance is released for a long period of time from immediately after administration, dispersibility is superior, the preparation can readily pass through a needle as an injection and the like.

**Brief Description of the Drawings**

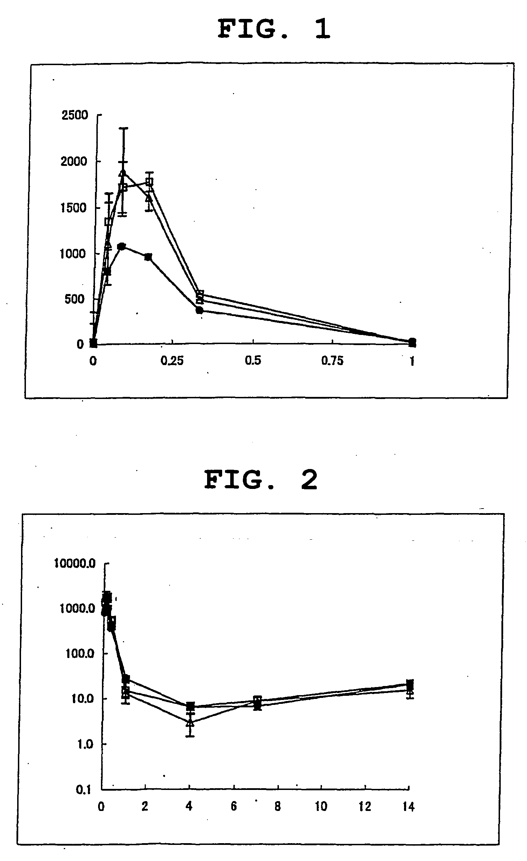

Fig. 1 is a graph obtained in Experimental Example 1, which shows serum hGH concentration profiles of immunosuppressed SD rats in 24 hours after administration of the sustained-release preparations obtained in Example 1, Comparative Example 1 and Comparative Example 2, wherein $-\bullet-$ shows a serum hGH concentration profile after administration of the sustained-release preparation obtained in Example 1, $-\square-$ shows a serum hGH concentration profile after administration of the sustained-release preparation obtained in Comparative Example 1, and $-\triangle-$ shows a serum hGH concentration profile after administration of the sustained-release preparation obtained in Comparative Example 2.

Fig. 2 is a graph obtained in Experimental Example 1, which shows serum hGH concentration profiles of immunosuppressed SD rats in 2 weeks after administration of the sustained-release preparations obtained in Example 1, Comparative Example 1 and Comparative Example 2, wherein $-\bullet-$ shows a serum hGH concentration profile after administration of the sustained-release preparation obtained in Example 1, $-\square-$ shows a serum hGH concentration profile after administration of the sustained-release preparation obtained in Comparative Example 1, and $-\triangle-$ shows a serum hGH concentration profile after administration of the sustained-release preparation obtained in Comparative Example 2.

**Best Mode for Embodying the Invention**

The dispersing agent in the present invention refers to a liquid medium used for injecting the sustained-release preparation as a suspension, and the like.

As the non water-soluble solvent in the dispersing agent of the present invention, vegetable oils such as soybean oil, sesame oil, corn oil, olive oil, camellia oil, rapeseed oil, peanut oil, cottonseed oil and the like, and the like are used. Of these, soybean oil is preferable.

As the aqueous solvent in the dispersing agent of the present invention, water, a mixture of water and a hydrophilic organic solvent, and the like are used. As the hydrophilic organic solvent, for example, ethanol can be mentioned. These hydrophilic organic solvents may be added in a proportion of 0 to about 50 parts by weight, preferably 0 to about 10 parts by weight, relative to 100 parts by weight of water. As the aqueous solvent in the present invention, water is preferably used.

The aqueous solvent in the present invention may contain one or more kinds selected from isotonicity agents (osmoregulators), surfactants, thickeners (viscosity agents), preservatives (stabilizers), soothing agents, local anesthetics, pH adjusting agents and the like.

As the isotonicity agent, for example, saccharides such as mannitol, sorbitol, glucose and the like, salts such as sodium chloride and the like and glycerol can be mentioned.

As the surfactant, a nonionic surfactant is preferable. As the nonionic surfactant, a higher alcohol ethylene oxide adduct, an alkylphenol ethylene oxide adduct, a fatty acid ethylene oxide adduct, a polyvalent alcohol fatty acid ester ethylene oxide adduct, a higher alkylamine ethylene oxide adduct, a fatty acid amide ethylene oxide adduct, an ethylene oxide adduct of fat and oil, a glycerol fatty acid ester, a fatty acid ester of pentaerythritol, an alkyl ether of polyvalent alcohol, a fatty acid amide of alkanolamine and the like are used.

Of the nonionic surfactants, for example, fatty acid esters of sorbitol and sorbitan, polyoxyethylene sorbitan fatty acid ester, polyethylene glycol fatty acid ester, sucrose fatty acid ester, polyoxyethylene castor oil (polyethoxylated castor oil), polyoxyethylene hydrogenated castor oil (polyethoxylated hydrogenated castor oil), polyoxyethylene polypropylene glycol copolymer, glycerol fatty acid ester, polyglycerol fatty acid ester and the like are preferably used. As the sorbitan fatty acid ester, sorbitan monostearate (product name: SS-10, Nikko Chemicals Co., Ltd.), sorbitan sesquioleate (product name: SO-15, Nikko Chemicals Co., Ltd.), sorbitan trioleate (product name: SO-30, Nikko Chemicals Co., Ltd.) and the like are particularly preferable. As the polyoxyethylene sorbitan fatty acid ester, polysorbate 20 (product name: TL-10, Nikko Chemicals Co., Ltd.), polysorbate 40 (product name: TP-10, Nikko Chemicals Co., Ltd.), polysorbate 60 (product name: TS-10, Nikko Chemicals Co., Ltd.), polysorbate 80 (product name: TO-10, Nikko Chemicals Co., Ltd.) and the like are particularly preferable. As the polyethylene glycol fatty acid ester, polyethylene glycol monolaurate (10E.O.) (product name: MYL-10, Nikko Chemicals Co., Ltd.) and the like are particularly preferable. As the sucrose fatty acid ester, sucrose palmitates (e.g., product name: S-1670, Mitsubishi-Kagaku Foods Corporation), sucrose stearates (e.g., product name: P-1670, Mitsubishi-Kagaku Foods Corporation) and the like are particularly preferable. As the polyoxyethylene castor oil (polyethoxylated castor oil), polyoxyethylene glycerol triricinoleate 35 (Polyoxy 35 Castor Oil, product name Cremophor EL or EL-P, BASF Japan Ltd.) and the like are particularly preferable. As the polyoxyethylene hydrogenated castor oil (polyethoxylated hydrogenated castor oil), polyoxyethylene hydrogenated castor oil 50, polyoxyethylene hydrogenated castor oil 60 and the like are particularly preferable. As the polyoxyethylene polyoxypropylene glycol copolymer, polyoxyethylene(160)polyoxypropylene(30)glycol (product name: Adeka Pluronic F-68, Asahi Denka Co., Ltd.) and the like are particularly preferable. As the glycerol fatty acid ester, glyceryl monostearate (MGS series, Nikko Chemicals Co., Ltd.) and the like are preferable. As the polyglycerol fatty acid ester, tetraglycerol monostearate (MS-310, Sakamoto Yakuhin Kogyo Co., Ltd.), decaglycerol monolaurate (Decaglyn 1-L, Nikko Chemicals Co., Ltd.) and the like are particularly preferable.

As the surfactant, polysorbate 80 (Tween 80), HCO-60 and the like are preferable.

A surfactant is preferably used in an amount that does not allow formation of a stable fine emulsion (e.g., lipid emulsion) of a non water-soluble solvent and an aqueous solvent, but can stabilize a dispersion system to the degree that, when a matrix containing a physiologically active substance is dispersed (e.g., emulsified by manual stirring) with a dispersing agent for injection of a sustained-release preparation, complete separation of a non water-soluble solvent from an aqueous solvent before injection, which impairs ready passage through a needle, is inhibited.

As the thickener, for example, water-soluble polysaccharides such as carboxymethylcellulose sodium, sodium alginate, sodium hyaluronate, dextran and the like, and the like can be mentioned.

As the preservative, for example, alkyl 4-hydroxybenzoates such as methyl 4-hydroxybenzoate, propyl 4-hydroxybenzoate and the like, and the like can be mentioned.

As the soothing agent, for example, benzyl alcohol and the like can be mentioned.

As the local anesthetic, for example, xylocaine hydrochloride, chlorobutanol and the like can be mentioned.

As the pH adjusting agent, for example, hydrochloric acid, acetic acid, sodium hydroxide, various buffers and the like can be mentioned.

These additives can be contained in a non water-soluble solvent, as long as the solubility, stability and the like do not suffer from inconvenience.

The volume of an aqueous solvent in the dispersing agent of the present invention is about 0.2 to 1000 times, preferably about 2 to 500 times, more preferably about 4 to 100 times, particularly preferably about 4 to 50 times, relative to the volume of a non water-soluble solvent.

The dispersing agent of the present invention is a bilayer dispersing agent containing one or more kinds of non water-soluble solvents and one or more kinds of aqueous solvents, and is used for dispersing a matrix containing a physiologically active substance. While conventional lipid emulsions also contain a non water-soluble solvent and an aqueous solvent, fine oil drops of the non water-soluble solvent form a stable homogeneous system with the aqueous solvent. In contrast, the dispersing agent of the present invention presents a lipid emulsion state for a certain time upon shaking (e.g., time sufficient for shaking to completion of injection of a dispersion), but cannot form a homogeneous system because the non water-soluble solvent and the water-soluble solvent are thermodynamically unstable and become a bilayer (the bilayer here means one that becomes a bilayer upon mixing, which is not necessarily prepared to form an interface between the non water-soluble solvent and the aqueous solvent, as long as it is inseparably used as a dispersing agent). In addition, conventional lipid emulsions can be produced by, for example, mixing an oil component (e.g., soybean oil), a phospholipid (e.g., egg yolk lecithin), water, other additives (e.g., glycerol) as necessary and the like, heating the mixture as necessary, and pulverizing sufficiently finely in a pressure spraying homogenizer such as Manton-Gaulin homogenizer and the like, a microfluidizer, an ultrasonic homogenizer and the like. Where necessary, it is further sterilized (sterilization by filtration, high pressure steam sterilization), filled and sealed in a container (e.g., ampoule) with a nitrogen gas. In contrast, a bilayer dispersing agent for a sustained-release preparation of the present invention can be produced by a production method for conventional solution injections. To be specific, for example, one or more kinds of additives selected from surfactants (e.g., polysorbate 80), thickeners (e.g., carboxymethylcellulose sodium) and the like are added as desired to a non water-soluble solvent (e.g., soybean oil), an aqueous solvent (e.g., water) and an isotonicity agent (e.g., mannitol), the mixture is filled and sealed in a container (e.g., ampoule) with, for example, a nitrogen gas, and subjected to a high pressure steam sterilization, whereby the dispersing agent can be produced.

Moreover, the sustained-release preparation of the present invention can be produced by (1) dispersing a matrix containing a physiologically active substance in a bilayer dispersing agent containing a non water-soluble solvent and an aqueous solvent, or (2) first dispersing a matrix containing a physiologically active substance in the above-mentioned non water-soluble solvent, and dispersing the dispersion by further adding the above-mentioned aqueous solvent. In the present specification, when "dispersing a matrix (or dispersion) in a dispersing agent or a non water-soluble solvent (or aqueous solvent)", its directionality is not limited, and a dispersing agent or a non water-soluble solvent (or aqueous solvent) may be added to a matrix (or dispersion) or a matrix (or dispersion) may be added to a dispersing agent or a non water-soluble solvent (or aqueous solvent).

The sustained-release preparation, which contains a matrix containing a physiologically active substance and the dispersing agent of the present invention, can be administered, for example, as a microcapsule or, by preparing various dosage forms using the microcapsule as a raw material, as parenteral preparations [non-intravascular injection (e.g., injections or preparations for implantation into muscle, hypodermis, organs and the like); intravascular injection or drip infusion and the like], or oral preparations (e.g., liquid preparations such as suspensions and the like). In the present specification, a "microcapsule" is used as a generic term referring to a solid microparticle carrier having a matrix structure, where the internal structural includes a homogenous matrix type carrier and what is called a reservoir type carrier (microcapsule in a narrow sense), and the external shape includes not only spheres (microsphere in a narrow sense) but also any shape acceptable as injections.

The sustained-release preparation of the present invention is particularly preferably used as an injection. For example, when the sustained-release preparation is a microcapsule, a practical sustained-release preparation for injection can be obtained by adding the dispersing agent of the present invention to give a suspension.

The physiologically active substance in the present invention includes, and not specifically limited to, for example, peptide compounds having physiological activity (hereinafter referred to "physiologically active peptide"), other antibiotics, antifungal agents, antihyperlipidemic agents, antitumor agents, antipyretic agents, analgesic agents, antiinflammatory agents, antitussive and expectorant agents, sedatives, muscle relaxants, anticonvulsants, antiulcer agents, antidepressants, antiallergic agents, cardiotonics, antiarrhythmic agents, vasodilators, hypotensive diuretics, antidiabetic agents, anticoagulants, hemostatic agents, antiplatelet agents, antituberculous agent, hormones, antinarcotics, bone resorption-suppressing agents, osteogenesis-accelerating agents, neovascularization suppressing agents and the like. Among these, peptide compound is specifically preferred.

The physiologically active peptide in the present invention includes various peptides or proteins, which have physiological activities useful for mammals and can be used clinically. The "physiologically active peptide" having a molecular weight as monomers of, for example, about 200 to 500,000, preferably molecular weight of about 1,000 to 500,000, is generally used. More preferably, a peptide having a molecular weight of 5,000 to about 500,000 is used. Typical activity of the physiologically active peptide includes hormone action. The physiologically active peptide may be a natural substance, a synthetic substance or a semi-synthetic substance, or may be a derivative or an analogue thereof. The action mechanism of the physiologically active peptide may be either agonistic or antagonistic.

As the physiologically active peptide of the present invention, for example, peptide hormones, cytokines, peptide neurotransmitters, hematopoietic factors, various growth factors, enzymes, antibodies peptide antibiotics, analgesic peptides and the like are used.

As the peptide hormones, for example, insulin, somatostatin, somatostatin derivatives (Sandostatin; see U. S. Pat. Nos. 4,087,390, 4,093,574, 4,100,117 and 4,253,998), growth hormones (GH), sodium diuretic peptides, gastrin, prolactin, adrenocorticotropic hormone (ACTH), ACTH derivatives (e.g., ebiratide and the like), melanocyte-stimulating hormone (MSH), thyrotropin-releasing hormone (TRH) and salts and derivatives thereof (see JP-A-50-121273 and JP-A-52-116465), thyroid-stimulating hormone (TSH), luteinizing hormone (LH), follicle-stimulating hormone (FSH), human chorionic gonadotropin (HCG), thymosin, motilin, vasopressin, vasopressin derivatives [desmopressin, see Folia Endocrinologica Japonica, Vol. 54, No. 5, pp. 676-691 (1978)], oxytocin, calcitonin, parathyroid hormone (PTH), glucagon, secretin, pancreozymin, cholecystokinin, angiotensin, human placental lactogen, glucagon-like peptide (GLP-1) and derivatives thereof (see JP-A-6-80584, JP-A-7-2695, EP658568, JP-A-8-245696, JP-A-8-269097, WO97/15296, WO97/31943, WO98/19698, WO98/43658, JP-A-10-511365, WO99/55310, JP-A-11-513983, CA2270320, WO99/64061, JP-A-11-514972, JP-A-2000-500505, WO2000/66138, WO2000/66142, WO2000/78333, JP-A-2001-11095, Tissue Eng. $\underline{7}$(1)35-44(2001), Diabetologia $\underline{43}$(10)1319-1328(2000), WO2000/34331, WO2000/34332, U.S. Pat. No. 6,268,343, US 2001011071 A, US 2001006943 A, EP0733644, WO2000/77039, WO99/43707, WO99/43341, WO99/43706, WO99/43708, WO99/43705, WO99/29336, WO2000/37098, EP0969016, U.S. Pat. No. 5,981,488, U.S. Pat. No. 5,958,909, WO93/25579, WO98/43658, EP0869135, U.S. Pat. No. 5,614,492, U.S. Pat. No. 5,545,618, U.S. Pat. No. 5,120,712, U.S. Pat. No. 5,118,666, WO95/05848, WO91/11457, EP0708179, WO96/06628, EP0658568, WO87/06941), metastin and derivatives thereof (see W02000/24890) and the like, are used. The peptide hormone preferably includes insulin and growth hormone and the like.

The cytokines include, for example, lymphokines, monokines and the like. The lymphokines include, for example, interferons (alpha, beta, gamma and the like) and interleukins (e.g., IL-2, 3, 4, 5, 6, 7, 8, 9, 10, 11, 12 and the like) and the like. The monokines include, for example, interleukin-1 (IL-1), tumor necrosis factor (TNF) and the like. The cytokine is preferably a lymphokine and the like, more preferably interferon and the like, especially preferably interferon-alpha.

The peptide neurotransmitters include, for example, substance P, serotonin, GABA and the like.

The hematopoietic factors include, for example, erythropoietin (EPO), colony stimulating factors (G-CSF, GM-CSF, M-CSF and the like), thrombopoietin (TPO), platelet-derived growth factor, megakaryocyte potentiator and the like.

The various growth factors include, for example, basic and acidic fibroblast growth factors (FGF) and their families (e.g., EGF, TGF-α, TGF-β, PDGF, acidic FGF, basic FGF, FGF-9 and the like), nerve growth factor (NGF) and its family (e.g., BDNF, NT-3, NT-4, CNTF, GDNF and the like), insulin-like growth factors (e.g. IGF-1, IGF-2 and the like), bone morphogenetic protein (BMP) and its family and the like.

The enzymes include, for example, superoxide dismutase (SOD), urokinase, tissue plasminogen activator (TPA), asparaginase, kallikrein and the like.

As the antibodies, for example, an anti-endothelin antibody (see JP-A-2-238894), an anti-endothelin-2 antibody (see JP-A-5-184388), an anti-endothelin-3 antibody (see JP-A-4-152894, JP-A-6-335397), an anti-pituitary adenylate cyclase activating polypeptide (PACAP) antibody (see WO91/14786), an anti-interleukin-2 antibody (see JP-A-62-135770), an anti-interferon-α antibody (see JP-A-61-67481), an anti-β-amyloid antibody (see WO94/17197), an anti-nerve growth factor (NGF) antibody (see JP-A-6-317587), an anti-nerve growth factor-2 (NGF-2) antibody (see JP-A-4-128298, JP-A-6-189787), an anti-basic fibroblast growth factor (bFGF) antibody (see JP-A-2-193), an anti-C5a receptor antibody (see JP-A-8-109200), an anti-metastin antibody (see WO03/27149) and the like are used. Additionally, a mouse anti-CD3 monoclonal antibody, a chimeric anti-gpIIb/IIIa monoclonal antibody, a mouse or chimeric anti-CD20 monoclonal antibody, a chimeric or humanized anti-interleukin-2 receptor monoclonal antibody, a humanized anti-erbB2 monoclonal antibody, a chimeric antitumor necrosis factor-α (TNF-α) monoclonal antibody, a humanized anti-F protein monoclonal antibody, a humanized anti-CD33 monoclonal antibody, a humanized anti-CD52 monoclonal antibody and the like, known as active ingredients of existing antibody pharmaceutical products can be also used.

The peptide antibiotics include, for example, polymixin B, colistin, gramicidin, bacitracin and the like.

The analgesic peptides include, for example, enkephalin, enkephalin derivatives (see U.S. Pat. No. 4,277,394 and EP 31567 A), endorphin, kyotorphin and the like.

Further, the physiologically active peptides include thymopoietin, dynorphin, bombesin, caerulein, thymostimulin, thymic humoral factor (THF), serum thymic factor (FTS) and derivatives thereof (see U.S. Pat. No. 4,229,438), other thymic factors [Igaku no Ayumi, Vol.125, No. 10, pp. 835-843 (1983)], neurotensin, bradykinin, and endothelin antagonistic peptides (see EP 436189 A, EP 457195 A and EP 496452 A, and JP-A-3-94692 and JP-A-3-130299) and the like.

The physiologically active peptides specifically preferably used for the present invention include luteinizing hormone releasing hormone (LH-RH) and a derivative having the similar action thereto, or LH-RH antagonistic substance, growth hormone, insulin and the like. Among these, growth hormone, especially human growth hormone, is preferred.

In the present invention, when the physiologically active peptide contains a metal, the metal contained in the physiologically active peptide may be removed previously, if desired. As the method for removing metal, known methods can be used. For example, insulin in amorphous form and containing the least amount of metal can be obtained by dialyzing a hydrochloric acidic aqueous solution of insulin to water or a solution of ammonium acetate and lyophilizing the dialysate.

Growth hormone originating from any species can be used, and is preferably human growth hormone. Further, although natural growth hormone extracted from the pituitary gland and the like can be used for the present invention, genetic recombinant GH (see JP-B-6-12996 and JP-B-6-48987) is preferred. The recombinant hGH having the same structure as that of a natural type without methionine at the N-terminal is more preferred. Such GH may be in the form of a metal salt, and the one containing substantially no metal is also used. The hGH having molecular weight of about 20K dalton as well as about 22K dalton (see JP-A-7-101877 and JP-A-10-265404) can be used. Furthermore, the derivatives of hGH or related protein thereof (see WO99/03887) can be used.

While the amount of the physiologically active substance in the sustained-release preparation of the present invention varies depending on the kind of the physiologically active substance and the like, it is, for example, generally about 0.1 to 50% (W/W), preferably about 0.2 to 30% (W/W), and more preferably about 0.5 to 20% (W/W) in the case of a physiologically active peptide.

The matrix in the present invention is a solid containing a physiologically active substance in the base (e.g., a biodegradable polymer), which optionally contains an additive, and is a unit that substantially controls sustained-release. Examples thereof include for example, a microcapsule, a rod for implantation and the like.

The biodegradable polymer used for the present invention includes polymers synthesized by catalyst-free dehydration polycondensation from one or more of α-hydroxycarboxylic acids (e.g., glycolic acid, lactic acid and the like), hydroxydicarboxylic acids (e.g., malic acid and the like), hydroxytricarboxylic acids (e.g., citric acid and the like) and the like, and having a free carboxyl group or mixtures thereof, poly-α-cyanoacrylic esters, polyamino acids (e.g., poly-γ-benzyl-L-glutamic acid and the like) and maleic anhydride polymers (e.g., a styrene-maleic acid copolymer and the like). These polymers may be a homopolymer or a copolymer. Polymerization type may be of the random, block or graft. When the above-mentioned α-hydroxycarboxylic acids, hydroxydicarboxylic acids and hydroxytricarboxylic acids have an optically active center in their molecules, they may be of the D-, L- or DL-configuration.

Among these polymers, a biodegradable polymer having a free terminal carboxyl group such as polymers synthesized from α-hydroxycarboxylic acids (e.g., glycolic acid, lactic acid and the like) (e.g., polylactic acid, lactic acid-glycolic acid copolymer and the like) and poly-α-cyanoacrylic acid esters are preferred.

The biodegradable polymer is more preferably a polymer synthesized from α-hydroxycarboxylic acids and the like, especially preferably lactic acid-glycolic acid copolymer and the like.

In the present specification, lactic acid-glycolic acid copolymer as well as homopolymers such as polylactic acid and polyglycolic acid are sometimes simply referred to as lactic acid-glycolic acid polymer.

When the biodegradable polymer used is a lactic acid-glycolic acid polymer (a lactic acid-glycolic acid copolymer or homopolymer), its composition ratio (mol %, lactic acid/glycolic acid) is preferably about 100/0 to about 40/60, more preferably about 85/15 to about 50/50.

The weight-average molecular weight of the lactic acid-glycolic acid polymer is preferably about 3,000 to about 50,000, more preferably about 3,000 to about 25,000, further more preferably about 5,000 to about 20,000.

The degree of dispersion (weight-average molecular weight/number-average molecular weight) of the lactic acid-glycolic acid polymer is preferably about 1.2 to about 4.0, more preferably about 1.5 to about 3.5.

Regarding weight-average molecular weight and degree of dispersion in the present specification, the former is the polystyrene reduced value determined by gel permeation chromatography (GPC) using 9 polystyrenes as reference substances with weight-average molecular weights of 120,000, 52,000, 22,000, 9,200, 5,050, 2,950, 1,050, 580 and 162, respectively, and the latter is the calculated value therefrom. The above determination is carried out using a GPC column KF804L x 2 (manufactured by Showa Denko K.K.) and an RI monitor L-3300 (manufactured by Hitachi, Ltd.) with chloroform as a mobile phase.

A biodegradable polymer having a free terminal carboxyl group is a polymer in which the number-average molecular weight based on terminal group determination and the number-average molecular weight based on GPC measurement above almost correspond with each other. The number-average molecular weight based on terminal group determination is calculated as follows:

About 1 to 3 g of the biodegradable polymer is dissolved in a mixed solvent of acetone (25 ml) and methanol (5 ml), and the solution is quickly titrated with a 0.05 N alcoholic solution of potassium hydroxide under stirring at room temperature (20°C) with phenolphthalein as an indicator to determine the carboxyl group in the solution; the number-average molecular weight based on terminal group determination is calculated from the following equation:

Number-average molecular weight based on terminal group

determination=$20000 \times A/B$

A: Weight mass (g) of biodegradable polymer

B: Amount (ml) of the 0.05 N alcoholic solution of potassium hydroxide added until titration end point is reached

While the number-average molecular weight based on terminal group determination is an absolute value, the number-average molecular weight based on GPC measurement is a relative value that varies depending on various analytical conditions (e.g., kind of mobile phase, kind of column, reference substance, slice width chosen, baseline chosen etc.); it is therefore difficult to have an absolute numerical representation of the latter. However, that both number-average molecular weights determined by GPC measurement and terminal group determination almost correspond with each other means, for example, that the number-average molecular weight based on terminal group determination falls within the range from about 0.5 to about 2 times, preferably from about 0.7 to about 1.5 times, of the number-average molecular weight based on GPC measurement in a polymer which is synthesized from α-hydroxycarboxylic acids.

For example, in the case of a polymer having a free terminal carboxyl group which is synthesized from one or more α-hydroxycarboxylic acids by catalyst-free dehydration polycondensation, the number-average molecular weight based on GPC measurement and the number-average molecular weight based on terminal group determination almost correspond with each other. On the other hand, in the case of a polymer having substantially no free terminal carboxyl group which is synthesized from a cyclic dimer by ring-opening polymerization using a catalyst, the number-average molecular weight based on terminal group determination is significantly (about 2 times or more) higher than that based on GPC measurement. This difference makes it possible to clearly differentiate a polymer having a free terminal carboxyl group from a polymer having no free terminal carboxyl group.

A lactic acid-glycolic acid polymer having a free terminal carboxyl group can be produced by a process known per se such as that described in JP-A-61-28521 (e.g., process by catalyst-free dehydration polycondensation reaction or dehydration polycondensation reaction in the presence of an inorganic solid acid catalyst).

The decomposition/disappearance rate of a lactic acid-glycolic acid polymer varies widely depending on composition ratio or weight-average molecular weight. A release duration of physiologically active substance can be extended (e.g., to about 6 months) by lowering the glycolic acid ratio or increasing the molecular weight, since decomposition/disappearance rate is usually delayed as the glycolic acid ratio decreases. Conversely, the release duration can be shortened (e.g., to about one week) by increasing the glycolic acid ratio or decreasing the molecular weight. To obtain a one week to two months type sustained-release preparation, it is preferable to use a lactic acid-glycolic acid polymer whose composition ratio and weight-average molecular weight are within the above-described ranges.

Therefore the composition of a biodegradable polymer used in the present invention is preferably selected according to the targeted kind of a physiologically active peptide, the desired sustained-release duration and the like. As the specific example, for example, when GH is used as a physiologically active peptide, a lactic acid-glycolic acid polymer is preferably used. The lactic acid-glycolic acid polymer is preferably a lactic acid-glycolic acid-copolymer having a lactic acid/glycolic acid composition ratio (mol %) of about 85/15 to about 50/50, more preferably about 75/25 to about 50/50. The weight-average molecular weight of the lactic acid-glycolic acid copolymer is preferably about 8,000 to about 20,000, more preferably about 10,000 to about 20,000. Further, the degree of dispersion (weight-average molecular weight/number-average molecular weight) of the lactic acid-glycolic acid polymer is about 1.2 to about 4.0, more preferably about 1.5 to about 3.5.

The lactic acid-glycolic acid polymer used can be produced by the known methods such as those described in the above publication and the like. The polymer is preferably the one that is produced by catalyst-free dehydration polycondensation. It is preferable that the lactic acid-glycolic acid polymer (PLGA) wherein the number-average molecular weight based on terminal group determination and the number-average molecular weight based on GPC measurement almost correspond with each other is used.

Further, two kinds of lactic acid-glycolic acid polymers differing in composition ratio and/or weight-average molecular weight may be used in an admixture of given ratio. The example is a mixture of lactic acid-glycolic acid copolymer wherein the composition ratio of lactic acid/glycolic acid (mol %) is about 75/25 and the weight-average molecular weight is about 10,000 and lactic acid-glycolic acid copolymer wherein the composition ratio of lactic acid/glycolic acid (mol %) is about 50/50 and the weight-average molecular weight is about 12,000. The preferred weight ratio of these copolymers in the mixture is about 25/75 to about 75/25, respectively.

The biodegradable polymer used in the present invention can be metal salts of the above mentioned biodegradable polymer. For example, various polyvalent metal salts of the biodegradable polymer and the like described in WO97/01331 can be used. Preferably, polyvalent metal salt of the lactic acid-glycolic acid polymer and the like (more preferably, zinc salt, calcium salt, magnesium salt and the like, further more preferably zinc salt and the like) can be used. The metal of the polyvalent metal salt is not particularly limited as long as it does not cause any adverse effect to a living body, and is exemplified by polyvalent metals such as bivalent metals (e.g., iron, zinc, copper, calcium, magnesium, aluminum, tin, manganese and the like), trivalent metals (e.g., iron, aluminum, manganese and the like), tetravalent metals (e.g., tin and the like) and the like.

In the present specification, not only the biodegradable polymer but also metal salt thereof is sometimes referred to as the biodegradable polymer. For example, a polyvalent metal salt of lactic acid-glycolic acid polymer is also sometimes referred to as lactic acid-glycolic acid polymer.

These polyvalent metal salts of the biodegradable polymer can be produced by the method described in WO97/01331 or similar methods thereto.

In case that polyvalent metal salt of the biodegradable polymer is a zinc salt, it can be produced by reaction of the biodegradable polymer and zinc oxide in an organic solvent.

Concerning the order of addition of biodegradable polymer and zinc oxide into organic solvent, zinc oxide in powder or suspension in organic solvent can be added into the solution of biodegradable polymer in organic solvent, or on the contrary, the solution of the biodegradable polymer in organic solvent can be added into the suspension of zinc oxide in organic solvent. Furthermore, after mixing both of the biodegradable polymer and zinc oxide in powder form, organic solvent can be added thereto.

The content of the biodegradable polymer contained in the sustained-release preparation of the present invention is generally about 30 to 99.9% (W/W), preferably about 60 to 97% (W/W), and more preferably about 70 to 90% (W/W).

In the production of the sustained-release preparation of the present invention, the organic solvent used to dissolve the biodegradable polymer preferably has a boiling point of not more than 120°C. The organic solvent includes, for example, halogenated hydrocarbons (e.g., dichloromethane, chloroform and the like), alcohols (e.g., ethanol, methanol and the like), ethyl acetate, acetonitrile and the like. These solvents may be used in a mixture of a suitable ratio. When one of the organic solvents is used solely, such as dichloromethane, ethyl acetate, acetonitrile and the like are preferred. When the organic solvents are used as a mixed solvent, such as a combination of halogenated hydrocarbons (e.g., dichloromethane, chloroform and the like) and alcohols (e.g., ethanol, methanol and the like) or acetonitrile is preferred. The mixing ratio (volume ratio) of the halogenated hydrocarbons and alcohols or acetonitrile is about 100:1 to about 1:1, and it is desirable to use a mixed solvent having a mixing ratio of preferably about 30:1 to about 2:1. Furthermore, while the concentration of the biodegradable polymer in a solution varies depending on the molecular weight, the kind of organic solvent and the like, it is, for example, about 0.01 to about 80% (W/W), preferably about 0.1 to about 70% (W/W), and more preferably about 1 to about 60% (W/W).

The sustained-release preparation in the present invention is a preparation obtained by forming a matrix and optionally adding an excipient (e.g., mannitol) thereto and treating (e.g., lyophilizing) it.

The initial release rate of the physiologically active substance in the present invention is the ratio of the amount of the physiologically active substance that has been released within one day after administration of the sustained-release preparation to an animal (rat) relative to the dose.

The matrix containing a physiologically active substance of the present invention is produced by removing the organic solvent from the S/O dispersion liquid in which the powder (S phase) obtained by lyophilizing a solution of physiologically active substance has been dispersed in the solution of the biodegradable polymer in the organic solvent (O phase), or removing the solvent from the W/O emulsion in which the aqueous phase (W phase) dissolving the physiologically active substance in water has been dispersed in the solution of biodegradable polymer in organic solvent (O phase), or removing the solvent from the solution in which a physiologically active substance and a biodegradable polymer have been dissolved in an organic solvent (O phase). The production method includes, for example, (a) in-water drying method (S/O/W method and W/O/W method or O/W method), (b) phase separation method (coacervation method) and (c) spray-drying method, or similar methods thereto and the like. Hereinafter, as a matrix containing a physiologically active substance, a production method of, for example, microcapsules is explained.

(a-1) In-Water Drying Method (S/O/W Method)

According to this method, at first a water-miscible organic solvent and/or a volatile salt is added to the aqueous solution of the physiologically active substance, and then, the physiologically active substance powder (S phase) is produced by lyophilization. On this occasion, in order to obtain fine powder, the salt concentration in the solution of physiologically active substance, for example, the ion concentration of an alkali metal (sodium, potassium, calcium and the like) is preferred to be low. For example, when the alkali metal is sodium, its ion concentration is preferably not more than about 10 $\mu$g/mL. The biodegradable polymer is then dissolved in the organic solvent, and the above physiologically active substance powder is added and dispersed into the resulting organic solvent solution. The ratio (ratio by weight) of the physiologically active substance and the biodegradable polymer is, for example, about 1:1000 to about 1:1, preferably about 1:200 to about 1:5, more preferably about 1:100 to about 1:5. Preferably, an external physical energy is applied to disperse the physiologically active substance powder uniformly into the organic solvent solution. As the method, for example, irradiation of ultrasonic wave, a turbine stirrer, a homogenizer and the like are used. The average particle size of the physiologically active substance in the organic solvent solution is preferably not more than about 10 $\mu$m, more preferably about 0.1 to about 5 $\mu$m, further more preferably about 0.5 to about 2 $\mu$m, and this is easily achieved by using the physiologically active substance powder obtained in the present invention. The average particle size of the physiologically active substance in the present invention means the value obtained by a laser diffraction particle size analyzer (SALD2000A: Shimadzu Corporation) using the dispersion liquid of the physiologically active substance in organic solvent such as dichloromethane prepared with a homogenizer. In this process, the physiologically active substance is added into a organic solvent such as dichloromethane at the concentration of about 20 to 100 mg/mL, and then stirred and dispersed using a homogenizer (e.g., Polytron (Kinematica)) at about 20,000 rpm for about 30 sec to 1 min. The resulting dispersion liquid is diluted adequately with the organic solvent into the measurable concentration range of the above laser diffraction particle size analyzer.

Further, the organic solvent dispersion liquid (S/O dispersion liquid) prepared as above mentioned is added into an aqueous solvent (W phase), and then the same external physical energy as above mentioned, for example, irradiation of ultrasonic wave, a turbine stirrer, a homogenizer and the like is applied to form the S/O/W emulsion. Then, the solvent of the oil phase is evaporated to produce the microcapsules. The volume of the water phase is selected from the volume of generally about 1 times to about 10,000 times, preferably about 2 times to about 5,000 times, more preferably about 5 times to about 2,000 times based on the volume of the oil phase.

An emulsifier can be added into the above external water phase. As the emulsifier, can be used any one which is capable of forming the generally stable S/O/W emulsion. The emulsifier includes, for example, anionic surfactants, nonionic surfactants, polyoxyethylene castor oil derivatives, polyvinylpyrrolidones, polyvinyl alcohols, carboxymethylcelluloses, lecithin, gelatin, hyaluronic acids and the like. These emulsifiers can be used in admixture thereof if desired. The concentration of the emulsifier in the external water phase is, preferably about 0.001% to 20% (W/W), more preferably about 0.01% to 10% (W/W), particularly preferably about 0.05% to 5% (W/W).

The microcapsules thus obtained are collected by centrifugation or filtration, washed with distilled water to remove the emulsifier and the like adhering to the surface of microcapsules, re-dispersed in distilled water, and lyophilized.

In the present invention, the water-miscible organic solvent which can be added to the aqueous solution of the physiologically active substance includes alcohols, and especially ethanol is preferably used alone. The amount of addition (concentration) of the water-miscible organic solvent to the aqueous solution of the physiologically active substance is about 0.03 to 0.5% (V/V), preferably about 0.06 to 0.25% (V/V), more preferably about 0.1 to 0.15% (V/V), by volume-ratio. By lyophilizing the aqueous solution of physiologically active substance obtained by adding the water-miscible organic solvent, the physiologically active substance powder can be prepared, which is easy to handle (superior in handling) and is very fine (has a small particle size).

The volatile salt to be added to the aqueous solution of the physiologically active substance in this method includes, for example, ammonium salt (e.g., ammonium acetate, ammonium bicarbonate, ammonium carbonate, ammonium chloride and the like, preferably ammonium acetate and the like). These salts can be used in admixture thereof in an appropriate ratio. The amount of addition of the volatile salt to the aqueous solution of the physiologically active substance is about 10 times to about 80 times mole, preferably about 10 times to about 70 times mole, more preferably about 15 times to about 70 times mole, further more preferably about 20 times to about 70 times mole, and the most preferably about 20 times to about 50 times mole by mole ratio. Similar to the case that the water-miscible organic solvent is added, by lyophilizing the aqueous solution of the physiologically active substance obtained by adding the volatile salt, the fine powder of the physiologically active substance can be prepared, which is easy to handle (superior in handling) and is very fine (has a small particle size).

In the present method, the water-miscible organic solvent and/or volatile salt added to the aqueous solution of the physiologically active substance can be used solely or in admixture thereof. When the water-miscible organic solvent and the volatile salt are used in combination, they can be added into the aqueous solution of the physiologically active substance in accordance with the above amount of addition respectively.

(a-2) In-Water Drying Method (W/O/W Method)

According to this method, water or suitable buffer is added to a physiologically active substance to give a solution of physiologically active substance (W phase). A biodegradable polymer is then dissolved in an organic solvent, and to this organic solvent solution is added the above-mentioned solution of physiologically active substance and is dispersed. The W/O emulsion thus obtained is added to an aqueous solvent (W phase). In the same manner as in the above-mentioned S/O/W method, microcapsules are obtained from W/O/W emulsion.

(a-3) In-Water Drying Method (O/W Method)

According to this method, a biodegradable polymer and a physiologically active substance are dissolved in an organic solvent. The organic solvent solution (O phase) is then added to an aqueous solvent (W phase), and in the same manner as in the above-mentioned S/O/W method, microcapsules are obtained from O/W emulsion.

(b) Phase Separation Method (Coacervation Method)

In this method, a coacervating agent is gradually added to the S/O dispersion liquid of (a-1) or the W/O emulsion of (a-2) or oil phase solution of (a-3) described above under stirring to precipitate and solidify microcapsules. The amount of the coacervating agent to be added is about 0.01 to about 1,000 times by volume, preferably about 0.05 to about 500 times by volume, especially preferably about 0.1 to about 200 times by volume of the dispersion liquid. Any coacervating agent can be used, as long as it is a polymeric, mineral oil or vegetable oil compound miscible with the organic solvent for dissolution of a biodegradable polymer and does not dissolve the biodegradable polymer used. Specifically, examples of such coacervating agents include silicone oil, sesame oil, soybean oil, corn oil, cottonseed oil, coconut oil, linseed oil, mineral oil, n-hexane, n-heptane and the like. Two or more of these can be used in combination. The microcapsules thus obtained are collected by filtration, washed repeatedly with heptane and the like to remove the coacervating agent. Further, washing is conducted in the same manner as in the above-mentioned (a), followed by lyophilization.

In the production of microcapsules by the in-water drying method or coacervation method, an antiaggregation agent can be added for preventing aggregation of particles. As examples of the antiaggregation agent, for example, mannitol, lactose, glucose, water-soluble polysaccharides such as starches (e.g., corn starch and the like), hyaluronic acid and its alkali metal salt; glycine, proteins such as fibrin and collagen; and inorganic salts such as sodium chloride, sodium hydrogen phosphate and the like can be used.

(c) Spray-Drying Method

In the present method, the S/O dispersion liquid of (a-1), the W/O emulsion of (a-2) or the oil phase solution of (a-3), described above, is sprayed via a nozzle into the drying chamber of a spray drier to volatilize the organic solvent in the fine droplets in a very short time to produce microcapsules. Such nozzles include, for example, a two-fluid nozzle, a pressure nozzle, a rotary disc and the like. It is also advantageous, if necessary, to spray an aqueous solution of the above-described antiaggregation agent via another nozzle simultaneously with spraying the above dispersion liquid in order to prevent aggregation of each microcapsule particles. The microcapsules thus obtained are further washed in the same manner as in the above-mentioned (a), and optionally heated (under reduced pressure, if desired) to remove water and organic solvents further.

As a method for preparation of the rod for implantation as the matrix containing a physiologically active substance in the present invention, a method comprising heating the mixture of the physiologically active substance and the base to the temperature not less than the glass transition temperature of the base and then molding in a mold or forming by extrusion and the like is exemplified. The shape thereof may be selected from any shapes in addition to rod type. Alternatively, the rod-shaped preparation for implantation can be prepared by pulverizing or microcapsulating the mixture of the physiologically active substance and the base by a certain method in advance, filling the mixture in a stainless tube or a Teflon (registered trademark) tube and compressing to mold the mixture. On this occasion, if necessary, the mixture may be heated to the temperature not less than the glass transition temperature of the base. For example, a rod-shaped preparation having an outer diameter of 2.0 mm can be obtained by filling the microcapsules obtained by the in-water drying method in a Teflon (registered trademark) tube having an inner diameter of 2.0 mm, heating at 60°C for 15 min, compressing the microcapsules with a rod having a diameter of 2.0 mm, cooling and forming the compressed microcapsules.

The sustained-release preparation of the present invention is preferably in the form of microparticles. This is because the sustained-release preparation does not provide undue pain to a patient when it is administered to said patient using an injection needle generally used for subcutaneous or intramuscular injection. The particle size of the sustained-release preparation is, for example, about 0.1 to 300 μm, preferably about 1 to 150 μm, specifically preferably about 2 to 100 μm as the mean particle diameter. Although varying depending on the kind of the physiologically active substance and the like, the content of the physiologically active substance in the sustained-release preparation of the present invention is, for example, in the case of physiologically active peptide, generally about 0.1 to 50% (W/W), preferably about 0.2 to 30% (W/W), and more preferably about 0.5 to 20% (W/W). The content of the biodegradable polymer contained in the sustained-release preparation of the present invention is generally about 30 to 99.9% (W/W), preferably about 60 to 97% (W/W), and more preferably about 70 to 90% (W/W).

The initial release rate of the physiologically active substance in the sustained-release preparation of the present invention [the release rate up to one day (24 hr) after administration] is preferably not more than about 50%, more preferably about 1% to about 30%, more preferably about 2% to about 20%, and the most preferably about 2% to about 15% of the dose administered. The initial release rate is obtained by obtaining the amount of initial release by applying the AUC (Area Under the Concentration-Time Curve) of the blood concentration up to 24 hrs after subcutaneous administration of the sustained-release preparation of the present invention to the linear calibration curve of dose and AUC that has been obtained from the AUC up to 24 hr after subcutaneous administration of the physiologically active substance solution, and then calculating the initial release rate.

When the sustained-release preparation is, for example, a microcapsule, the particle size of the microcapsule for an injectable suspension may be within the range satisfying the requirements as to the degree of dispersion, and the property to pass through a needle. For example, the particle size is within the range of about 0.1 to about 300 μm, preferably about 1 to about 150 μm, more preferably about 2 to about 100 μm, as the average particle size.

Methods for preparing the above microcapsule as a sterile preparation include, but are not limited to, a method in which the entire production process is sterile, a method for sterilization in which the gamma rays are irradiated and a method in which an antiseptic is added.

The sustained-release preparation of the present invention is less toxic and can be safely used in mammals (e.g., human, bovine, pig, dog, cat, mouse, rat, rabbit and the like).

Indication of the sustained-release preparation varies variously depending on the physiologically active substance used. The sustained-release preparation is useful to prevent or treat diabetes when the physiologically active substance is insulin; viral hepatitis (e.g., hepatitis C, HBe antigen-positive active hepatitis and the like) and cancer (e. g., renal carcinoma, multiple myeloma and the like) when the physiologically active substance is interferon-α; anemia (e.g., anemia during dialysis of kidney and the like) when the physiologically active substance is erythropoietin; neutropenia (e.g., in cancer therapy) and infections when the physiologically active substance is G-CSF; cancer (e.g., hemangioendothelioma and the like) when the physiologically active substance is IL-2; fracture, wound (e.g., bedsore and the like), periodontitis and gastrointestinal ulcer when the physiologically active substance is FGF; thrombocytopenia when the physiologically active substance is FGF-9; senile dementia and neuropathy when the physiologically active substance is NGF; thrombosis when the physiologically active substance is TPA; and cancer when the physiologically active substance is tumor necrosis factor. Further, the GH-containing sustained-release preparation can be applied to Turner's syndrome, chronic renal failure, achondroplasia (cartilage dystrophia), and adult hypopituitarisin (adult GHD), exhaustive diseases such as AIDS and the like, as well as GH-deficient short stature, based on growth hormone action of GH. Further, it is reported that GH is applied to diseases such as Down syndrome, Silver syndrome, dysostosis and juvenile chronic arthritis to provide excellent therapeutic effects, therefore the GH-containing sustained-release preparation can be applied to these diseases. The GH-containing sustained-release preparation is also useful to prevent or treat congestive heart-failure and the like. The other indications to which the GH-containing sustained-release preparation can be applied include hematogenesis in organ transplantation or medication for an AIDS patient, improvement of hypoalimentation, renal anemia, angina pectoris, hyperlipidemia, obesity, acceleration of treatment for burn, wound or ulcer, early recovery from surgical invasion (operation, lesion)/postoperation, sepsis, prevention of fracture due to osteoporosis, early recovery of postoperative muscular power of a fracture patient due to osteoporosis, amyotropic lateral sclerosis (ALS), decubitus and the like. Furthermore, it is expected to have effects as an antiaging agent aimed at improving the quality of life (QOL) for frail aged persons, or effects for suppressing the development of or improving neurodegenerative diseases (Alzheimer's disease, Parkinson's disease, cerebrovascular disease and the like) due to the nerve protective effect of hGH. By preparing GH into a sustained-release preparation, medicinal effects superior to those of a daily GH subcutaneous injection can be obtained for these indications.

Although varying depending on the kind and content of the physiologically active substance, duration of the release, target disease, subject animal and the like, the dose of the sustained-release preparation may be any amount as long as the effective concentration of the physiologically active substance in the body is maintained. For example, when the sustained-release preparation is the one designed for two week release, the dose of the physiologically active substance can be suitably chosen from the range of preferably about 0.0001 to about 10 mg/kg body weight, more preferably about 0.05 to about 1 mg/kg body weight, per an adult. The administration frequency of the sustained-release preparation can be suitably chosen from once a week, once every two weeks, once a month, once every two months and the like, depending on the kind and content of the physiologically active substance, the dosage form, duration of the release, target disease, subject animal and the like. One week-release to two months-release type sustained-release preparation is preferred and one week-release to one month-release type sustained-release preparation is more preferred.

When the physiologically active substance as an active ingredient of the sustained-release preparation is, for example, insulin, the dose for an diabetic adult is suitably chosen from the range of usually about 0.001 to about 1 mg/kg body weight, preferably about 0.01 to about 0.2 mg/kg body weight, as an effective ingredient, and an administration of once a week is preferred.

When the physiologically active substance as an active ingredient of the sustained-release preparation is GH, the dose may be any amount as long as the effective concentration of GH in the body is maintained, although varying depending on the kind and content of GH, duration of the release, target disease, subject animal and the like. In the treatment of the above described diseases, when the sustained-release preparation is a two week-release type preparation, the dose of GH can be suitably chosen from the range of about 0.01 to about 5 mg/kg body weight (about 0.03 to about 15 IU/kg body weight), more preferably about 0.05 to about 1 mg/kg body weight (about 0.15 to about 3 IU/kg body weight), per an infant or an adult for safe administration. The administration frequency can be suitably chosen from once a week, once every two weeks, once a month and the like, depending on the content of GH, the dosage form, duration of the release, target disease, subject animal and the like. One week-release to two months-release type sustained-release preparation is preferred, and one week-release to one month-release type sustained-release preparation is more preferred.

The sustained-release preparation is preferably stored at ordinary temperature or in a cold place. More preferably, the sustained-release preparation is stored in a cold place. The "ordinary temperature" and the "cold place" are defined in the Pharmacopoeia of Japan. Namely, the "ordinary temperature" means 15 to 25°C, and the "cold place" means a temperature of not more than 15°C. In the "cold place", a temperature of 2 to 8°C is particularly preferred.

Hereinafter the present invention is explained more specifically with referring to the Reference Example, Example, Comparative Examples and Test Example, which do not limit the present invention.

**Reference Example 1**

To an aqueous solution of recombinant hGH (hGH concentration=2 mg/ml) was added ammonium acetate (20-fold mol equivalent) and the mixture was frozen rapidly and dried in vacuo to give hGH powder. A lactic acid-glycolic acid copolymer (lactic acid/glycolic acid=65/35, viscosity=0.160 dL/g; 69.375 g) and zinc oxide (0.375 g) were dissolved in dichloromethane (135 g). To the organic solvent solution was added the above-mentioned hGH powder (11.25 g) and the mixture was atomized with Minimixer (Tokushu Kika Kogyo Co., Ltd.). This S/O dispersion was added to a 0.1% aqueous solution of polyvinyl alcohol (30 L) and the mixture was stirred and emulsified using a homomixer. The obtained emulsion was stirred at room temperature for 3 hr to evaporate dichloromethane and centrifuged (about 1,800 rpm) to collect microcapsules. To the microcapsules was added D-mannitol (9 g) and the mixture was freeze-dried to give hGH-containing microcapsules.

**Comparative Example 1**

(1) Production of dispersion medium

Mannitol (5 g), carboxymethylcellulose sodium (0.5 g) and polysorbate 80 (0.1 g) were dissolved in distilled water for injection, and the solution was made up to 100 mL with distilled water for injection. The obtained solution was filtered through a filter having a pore size of 0.45 µm to give a dispersion medium for microcapsule injection.

(2) Production of sustained-release preparation

The hGH-containing microcapsule obtained in Reference Example 1 was dispersed in the dispersion medium obtained in the above-mentioned (1) to give a sustained-release preparation.

**Comparative Example 2**

The hGH-containing microcapsule obtained in Reference Example 1 was dispersed in INTRAFAT (0.75 mL) to give a sustained-release preparation.
INTRAFAT is a lipid emulsion containing a soybean oil (10 w/v%), and an INTRAFAT injection manufactured by Nihon Pharmaceutical Co., Ltd. was used.

**Example 1**

The hGH-containing microcapsule obtained in Reference Example 1 was dispersed in a soybean oil (0.075 mL) and the obtained dispersion was further dispersed in a dispersion medium (0.675 mL) obtained in Comparative Example 1(1) to give a sustained-release preparation.

**Test Example 1**

Using the sustained-release preparations obtained in Example 1, and Comparative Examples 1 and 2, the following test was performed.

(1) In Vivo Release in Rat

Microcapsule (6 mg/rat in hGH amount) was subcutaneously administered to immunosuppressed SD rats (male, 6-week-old). The blood was drawn with the lapse of time, hGH concentration in serum was measured by radioimmunoassay (Ab beads HGH, Eiken Chemical Co., Ltd.), and hGH sustained-release was evaluated. The immunosuppressed SD rats were prepared by subcutaneous administration of PROGRAF (Fujisawa Pharmaceutical Co., Ltd.) 3 days before microcapsule administration (0.4 mg/rat), and on administration, 4 days later and 7 days later (0.2 mg/rat each time). The results are shown in Fig. 1 and Fig. 2.

(2) Initial Release Rate

An aqueous solution of hGH (concentration 5, 10, 20 mg/kg) was subcutaneously administered to immunosuppressed SD rats (male, 6-week-old). The blood was drawn with the lapse of time for 24 hr, hGH concentration in serum was measured by radioimmunoassay (Ab beads HGH, Eiken Chemical Co., Ltd.), and AUC (Area Under the Concentration) at each concentration was determined to give a linear calibration curve of dose and AUC.
In addition, microcapsule (12 mg/rat in hGH amount) was subcutaneously administered to immunosuppressed SD rats (male, 6-week-old). The blood was drawn with the lapse of time for 24 hr, hGH concentration in serum was measured by radioimmunoassay (Ab beads HGH, Eiken Chemical Co., Ltd.), and AUC of each microcapsule was determined. By applying the obtained AUC to the linear calibration curve of dose and AUC to give an initial release amount, and the initial release rate was calculated.
The initial release rate of the sustained-release preparation of Example 1 was 16.9%, and the initial release rates of the sustained-release preparations of Reference Examples 1 and 2 were 27.6% and 26.1%, respectively.
As is clear from the above results, the sustained-release preparation of Example 1 showed small initial release (amount released up to day 1 of administration), and high sustained concentration (amount released after day 1 up to 2 weeks after administration). That is, by the use of, as a dispersing agent, a non water-soluble solvent and an aqueous solvent without stable emulsification, suppression of the initial release of a sustained-release preparation and sustention of a high blood hGH concentration for 2 weeks were achieved.

**Industrial Applicability**

According to the present invention, when a sustained-release preparation containing a non water-soluble solvent and an aqueous solvent as a dispersing agent for a microcapsule containing a physiologically active substance is subcutaneously administered, a sustained-release preparation having very superior clinical characteristics as a medicine, in which the initial release of a physiologically active substance immediately after administration is remarkably suppressed, a constant amount of the physiologically active substance is released after administration over a prolonged period of time, dispersibility is superior and the preparation can readily pass through a needle as an injection, can be obtained.

**Claims**

1.  A bilayer dispersing agent for a sustained-release preparation, which comprises a non water-soluble solvent and an aqueous solvent.

2.  The dispersing agent of claim 1, wherein the non water-soluble solvent is a vegetable oil.

3.  The dispersing agent of claim 2, wherein the vegetable oil is a soybean oil.

4.  The dispersing agent of claim 1, wherein the aqueous solvent is water.

5.  The dispersing agent of claim 1, wherein the volume of the aqueous solvent is about 0.2 to about 1000 times the volume of the non water-soluble solvent.

6.  The dispersing agent of claim 1, which comprises an isotonicity agent.

7.  The dispersing agent of claim 6, wherein the isotonicity agent is mannitol, sorbitol, sodium chloride, glucose or glycerol.

8.  The dispersing agent of claim 1, which comprises a surfactant.

9.  The dispersing agent of claim 8, wherein the surfactant is a nonionic surfactant.

10. The dispersing agent of claim 1, which comprises a thickener.

11. The dispersing agent of claim 10, wherein the thickener is carboxymethylcellulose, sodium alginate or sodium hyaluronate.

12. The dispersing agent of claim 1, which comprises a preservative.

13. The dispersing agent of claim 12, wherein the preservative is alkyl 4-hydroxybenzoate.

14. The dispersing agent of claim 1, which is used for injection.

15. A sustained-release preparation comprising a matrix containing a physiologically active substance and the dispersing agent of claim 1.

16. The sustained-release preparation of claim 15, wherein the physiologically active substance is a physiologically active peptide.

17. The sustained-release preparation of claim 16, wherein the physiologically active peptide has a molecular weight of about 200 to about 500,000.

18. The sustained-release preparation of claim 16, wherein the physiologically active peptide has a molecular weight of about 5,000 to about 500,000.

19. The sustained-release preparation of claim 16, wherein the physiologically active peptide is a hormone, a cytokine, a hematopoietic factor, a growth factor, an enzyme or an antibody.

20. The sustained-release preparation of claim 16, wherein the physiologically active peptide is a human growth hormone.

21. The sustained-release preparation of claim 15, wherein a base for the matrix is a biodegradable polymer.

22. The sustained-release preparation of claim 21, wherein the biodegradable polymer is a homopolyer or copolymer of $\alpha$-hydroxycarboxylic acids or a mixture thereof.

23. The sustained-release preparation of claim 21, wherein the biodegradable polymer is a copolymer having a composition ratio of lactic acid/glycolic acid of about 100/0 to about 40/60 mol %.

24. The sustained-release preparation of claim 21, wherein the biodegradable polymer is a homopolymer of lactic acid.

25. The sustained-release preparation of claim 21, wherein the biodegradable polymer has a weight-average molecular weight of about 3,000 to about 50,000.

26. The sustained-release preparation of claim 15, wherein the matrix is a microcapsule.

27. A production method of a sustained-release preparation, which comprises dispersing a matrix containing a physiologically active substance in the dispersing agent of claim 1.

28. A production method of a sustained-release preparation, which comprises dispersing a matrix containing a physiologically active substance in a non water-soluble solvent, and further dispersing the obtained dispersion in an aqueous solvent.

29. A sustained-release preparation obtained by the method of claim 27 or 28.

# FIG. 1

# FIG. 2

<div align="center">

**INTERNATIONAL SEARCH REPORT**

</div>

| | International application No. |
| --- | --- |
| | PCT/JP2004/001760 |

| A. CLASSIFICATION OF SUBJECT MATTER |
| --- |
| Int.Cl$^7$ A61K47/44, 9/50, 9/66, 47/02, 47/10, 47/14, 47/26, 47/34, 47/36, 47/38, 38/00, 38/22, 38/27, A61P43/00 |
| According to International Patent Classification (IPC) or to both national classification and IPC |

| B. FIELDS SEARCHED |
| --- |
| Minimum documentation searched (classification system followed by classification symbols) |
| Int.Cl$^7$ A61K47/44, 9/50, 9/66, 47/02, 47/10, 47/14, 47/26, 47/34, 47/36, 47/38, 38/00, 38/22, 38/27, A61P43/00 |

| Documentation searched other than minimum documentation to the extent that such documents are included in the fields searched |
| --- |
| Jitsuyo Shinan Koho          1922–1996    Toroku Jitsuyo Shinan Koho    1994–2004 |
| Kokai Jitsuyo Shinan Koho    1971–2004    Jitsuyo Shinan Toroku Koho    1996–2004 |

| Electronic data base consulted during the international search (name of data base and, where practicable, search terms used) |
| --- |
| CAPLUS(STN), MEDLINE(STN), BIOSIS(STN), EMBASE(STN) |

| C. DOCUMENTS CONSIDERED TO BE RELEVANT | | |
| --- | --- | --- |
| Category* | Citation of document, with indication, where appropriate, of the relevant passages | Relevant to claim No. |
| X<br>Y | WATNASIRICHAIKUL, Suchat et al., In-vitro release and oral bioactivity of insulin in diabetic rats using nanocapsules dispersed in biocompatible microermlsion Journal of Pharmacy and Pharmacology, 2002, Vol.54, pages 473 to 480; full text; particularly, page 475, right column; Figs. 2, 3 | 1-20,26-29<br>21-25 |
| X<br>Y | JP 2002-534459 A (LG. Chemical Ltd.), 15 October, 2002 (15.10.02), Full text; particularly, example 36 & WO 00/41682 A1. | 1-20,27-29<br>21-26 |
| Y | JP 8-217691 A (Takeda Chemical Industries, Ltd.), 27 August, 1996 (27.08.96), Full text; particularly, Claims 1 to 24; example 4 (Family: none) | 1-29 |

| ☐ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 19 April, 2004 (19.04.04) | 11 May, 2004 (11.05.04) |

| Name and mailing address of the ISA/ | Authorized officer |
| --- | --- |
| Japanese Patent Office | |
| Facsimile No. | Telephone No. |

Form PCT/ISA/210 (second sheet) (January 2004)